# EUROPEAN PATENT SPECIFICATION

(11) **EP 1 853 924 B1**
(45) Date of publication and mention of the grant of the patent: **22.07.2009**
(21) Application number: 05744943.1
(22) Date of filing: 27.01.2005
(51) Int. Cl.: G01N 33/74

(54) **A METHOD FOR MEASURING PROINSULIN AND C-PEPTIDE AND A KIT THEREFOR**
VERFAHREN ZUR MESSUNG VON PROINSULIN UND C-PEPTID SOWIE KIT DAFÜR
METHODE DE MESURE DE LA PROINSULINE ET D'UN PEPTIDE C ET KIT CORRESPONDANT

(43) Date of publication of application: 14.11.2007
(73) Proprietor: VRIJE UNIVERSITEIT BRUSSEL, 1050 Brussel (BE); OPUS NV, 1761 Borchtlombeek (BE)
(72) Inventor: DE PAUW, Pieter, B-1730 Asse (BE)
(74) Representative: Brants, Johan P.E.
(86) International application number: PCT/EP2005/000785
(87) International publication number: WO 2006/079354

(56) References cited:
- EP-A- 0 484 961
- EP-A- 1 191 337
- WO-A-98/59246
- RODER M E ET AL: "Disproportionately elevated proinsulin levels precede the onset of insulin-dependent diabetes mellitus in siblings with low first phase insulin responses. The Childhood Diabetes in Finland Study Group." THE JOURNAL OF CLINICAL ENDOCRINOLOGY AND METABOLISM. DEC 1994, vol. 79, no. 6, December 1994 (1994-12), pages 1570-1575, XP002349998 ISSN: 0021-972X cited in the application
- SASAKUMA F ET AL: "[A method for determination of proinsulin levels in serum using both insolubilized anti-insulin antibody and anti-C-peptide antibody]" RINSHO BYORI. THE JAPANESE JOURNAL OF CLINICAL PATHOLOGY. JUL 1992, vol. 40, no. 7, July 1992 (1992-07), pages 769-774, XP008053759 ISSN: 0047-1860
- ZILKER T R ET AL: "Determination of proinsulin intermediates by means of an immunoradiometric method using polyclonal antibodies" HORMONE AND METABOLIC RESEARCH 1988 GERMANY, vol. 18, no. SUPPL., 1988, pages 48-55, XP008053752 ISSN: 0018-5043
- SASAKUMA F ET AL: "[An improved method for determination of C-peptide levels in serum: pretreatment of test samples with anti-insulin antibody insolubilized with magnetic particles]" RINSHO BYORI. THE JAPANESE JOURNAL OF CLINICAL PATHOLOGY. FEB 1994, vol. 42, no. 2, February 1994 (1994-02), pages 183-188, XP008053760 ISSN: 0047-1860

## Description

### Field of the Invention

The present invention relates to methods to determine the concentrations of proinsulin and of C-peptide in a sample and to kits for use in such methods.

### Background to the Invention

The pancreatic peptide hormone insulin plays a major role in the regulation of glucose metabolism, generally promoting the cellular utilization of glucose. It is also an important regulator of protein and lipid metabolism.

In response to extracellular stimuli like glucose or glucagon, insulin is produced in beta cells of the islets of Langerhans from an 11,5 kDa precursor polypeptide, preproinsulin, which is rapidly transformed into proinsulin, a 9 kDa peptide. Proinsulin contains the A and B chains of insulin, joined by a connecting C-peptide. Proinsulin is transported to the Golgi apparatus where it is directed towards nascent secretary granules, in which proinsulin is converted to mature insulin by removal of the C-peptide. This conversion is achieved to a large extent before secretion - in normal beta cells, the proportion of proinsulin over insulin or C-peptide is less than 5%. Insulin and C-peptide are secreted in equimolar quantities when beta granules fuse with the plasma membrane.

The conversion of proinsulin into C-peptide and insulin requires prohormone convertases 2 and 3 (PC2 and PC3). PC2 cleaves proinsulin at A/C junction and PC3 at B/C junction, forming respectively the '65-66 split' and the '32-33 split' intermediates. These intermediates are further processed to 'des 64, 65' and 'des 31, 32' forms by carbopeptidase H. When cleavage at both sites is complete, C-peptide and insulin are produced.

Partially processed forms of proinsulin are also found in circulation, with in majority the 'des 31, 32' intermediate, followed by the 'des 64, 65' intermediate.

Circulating levels of proinsulin, insulin and C-peptide depend on the rate of secretion into the blood stream and clearance from it. In non-diabetic, healthy individuals, insulin has a half-life of about 4 min, proinsulin of 30 min and C-peptide of 33 min. The insulin level measured in peripheral plasma of 1418 fasting healthy individuals is 117 ± 63 pmol/L, whereas the proinsulin level is 4.78 ± 2.28 pmol/L (Grill et al. Am J Epidemiol 2002; 155:834-41). Taking into account the slower clearance ratio of C-peptide compared to insulin, the ratio of proinsulin to C-peptide (hereinafter "proinsulin/C-peptide ratio") may be considered as a more stable parameter than the proinsulin/insulin ratio, ranging between 0.37% - 1.3% in seemingly healthy individuals (Snorgaard et al. Diabetologia 1990; 33:36-42). In some cases where rapid responses of the pancreas to stimuli like glucose or glucagon are studied, the proinsulin/insulin ratio is preferably considered because of its higher response-to-clearance ratio (Tura A et al. Am J Physiol Endocrinol Metab 2003; 285:E155-E162). In patients treated with recombinant human insulin, only C-peptide can be considered as parameter for the function of the pancreas, because no distinction can be made between administered insulin and autologous insulin.

The proinsulin/C-peptide ratio can be considered as a marker for the functional state of the pancreatic beta cells: on one hand, lower insulin production - and thus lower C-peptide - is a direct indication of lowered functionality, on the other hand, higher proinsulin levels indicate that conversion to insulin and C-peptide is occurring less efficiently. Smaller than normal quantities of prohormone convertases may lead to higher levels of prohormones in malfunctioning islet cells (Tomita. Pancreas 2001; 23:172-6). The proinsulin/C-peptide ratio has been proposed as an indicator of the capacity of insulin secretion (Choi et al. Horm Metab Res 1999; 31:267-70).

Beta cells cultured in high glucose released more proinsulin and less insulin. Arginine also had a similar effect on secretion of proinsulin. It has been proposed that chronic glucose stimulation recruits all beta cells into an active state and that extracellular proinsulin levels may serve as an indicator of maximal insulin-biosynthetic capacity (Hostens et al. J Clin Endocrinol Metab 1999; 84:1386-90). Exposure of human beta cells to cytokine combinations, such as IL-beta plus IFN-gamma, also caused disproportionately elevated proinsulin levels in the extra cellular medium. The delay in proinsulin conversion could be attributed to lower expression of PC2 and PC3 convertases (Hostens et al. J Clin Invest 1999; 104:67-72).

In certain cases hyperproinsulinemia occurs. For example, release of incompletely processed proinsulin is the cause of disproportionate proinsulinemia in NIDDM (Kahn and Halban. Diabetes 1997; 46:1725-32), and reflects the degree of impaired beta cell secretory capacity in patients - a study showed that fasting proinsulin/insulin ratio was significantly elevated in type 2 diabetics compared to control subjects; although insulin level was similar, the ratio correlated inversely with maximal beta cell secretory capacity (Roder et al. J Clin Endocrinol Metab 1998; 83:604-8).

Also in type 1 diabetes, fasting proinsulin concentration and proinsulin/C-peptide ratio were both higher at the time of diagnosis in patients, compared to control subjects, respectively 5.1 pmol/L vs. 2.5 pmol/L and 2.6 % vs. 0.7 % (Snorgaard et al. Diabetologia 1990; 33:36-42).

Elevated fasting proinsulin levels were found not only in siblings of patients with type 1 diabetes (Hartling et al. Eur J Endocrinol 1997; 137:490-4), but also in their parents, suggesting that this may be a feature common to first-degree relatives of type 1 diabetic patients (Spinas et al. Diabetes Care 1992; 15:632-7.36).

A change in the proinsulin levels may precede abnormalities of insulin response to a glucose challenge and may therefore be an indicator of beta-cell dysfunction before onset of type 1 diabetes (Heaton et al. Diabetologia 1988; 31:182-4.). Decreased first phase insulin secretion during intravenous glucose tolerance testing (IVGTT) can be observed before onset (Roder et al. J Clin Endocrinol Metab 1994; 79:1570-5; Robert et al. Diabetes Care 1991; 14:718-23.). However; reproducibility of IVGTT is low-with high intra-individual coefficients of variation ranging between 21% (Hedstrand et al. Scand J Clin Lab Invest 1975; 35:331-7) and 32% (Sparkes et al. Am J Vet Res 1996; 57:1294-8). A prospective study showed that siblings of type 1 diabetes patients who had a low first phase of insulin release and who developed type 1 diabetes later had a disproportionately high proinsulin/C-peptide ratio during the pre-diabetic phase, suggesting that an elevated ratio may be an additional marker for later development of IDDM (Roder et al. J Clin Endocrinol Metab 1994; 79:1570-5). Recently, preservation of C-peptide secretion in subjects at high risk of developing type diabetes was found in the non-progressors, as opposed to progressors (Schatz et al. Pediatr Diabetes 2004; 5:72-9).

The observed changes mentioned above contribute to a higher proinsulin/C-peptide ratio in the preclinical phase of type 1 diabetes. That is why the proinsulin/C-peptide ratio is proposed as a predictive marker, especially in the short term period before onset. Proinsulin/C-peptide ratio measured at random (i.e. not fasting) occasion may provide a dynamic test, the screening of which may be simpler and more cost effective than of a complete IVGTT (in-vitro glucose tolerance test) or OGTT (oral glucose tolerance test). This is certainly so when compared to a clamping procedure, the gold standard for measuring pancreas functionality as described by DeFronzo RA et al. (Am J Physiol 1979; 237:E214-E223).

The proinsulin/C-peptide ratio might also become an interesting parameter in the prevention of other diseases. For example, relatively high proinsulin levels precede acute myocardial infarction in a non-diabetic population (Lindahl et al. Metabolism 1999; 48:1197-202); and proinsulin is an independent predictor of coronary heart disease (Zethelius et al. Circulation 2002; 105:2153-8).

As illustrated above, proinsulin/C-peptide ratio is a useful biological marker in assessing the functional state of the pancreatic beta cells and in predicting the risk for developing select disorders, such as type 1 diabetes. This notion is confirmed by the observation of statistically significant differences in the proinsulin/C-peptide ratio between populations of individuals having distinct clinical pictures or distinct risks for developing a disease. However, there remains a considerable overlap in individual proinsulin/C-peptide ratios between such different populations when measured with current techniques. This limits the application of the proinsulin/C-peptide ratio as a predictive marker in individual prognosis.

The overlap may be partially caused by analytical errors inherent to the current methods of determining the ratio. Up till now, in all publications considering the proinsulin/C-peptide or proinsulin/insulin ratios (see above references), the ratios are estimated from concentrations of proinsulin and C-peptide or proinsulin and insulin, respectively, which are measured by separate immunoassay methods, performed in separate reaction compartments (Clark PM. Assays for insulin, proinsulin(s) and C-peptide. Ann Clin Biochem 1999; 36 (Pt 5):541-64). In such case, the analytical errors of the separate methods cumulate when determining the ratio.

An aim of the present invention is therefore to provide an improved method that allows determining the proinsulin/C-peptide ratio or proinsulin/insulin ratio with higher precision than can be achieved by currently available approaches.

Further, Røder et al. 1994 (J Clin Endocrinol Metab 79(6): 1570-5) measures levels of proinsulin immunoreactivity (PIM), insulin immunoreactivity (IRI) and C-peptide using separate methods.

EP 1 191 337 describes an immunoassay for solely measuring C-peptide in a sample using two different C-peptide antibodies not recognising the same epitope.

Zilker et al. 1988 (Horm Metab Res Suppl 18: 48-55) discloses an immunoradiometric method for solely measuring proinsulin using an insulin and a C-peptide antibody.

### Summary of the Invention

As described above, the proinsulin/C-peptide ratio is a useful biological marker in assessing the functional state of the pancreatic beta cells and in predicting the risk for developing select disease phenotypes, such as type 1 diabetes. The inventors further discovered that in a population with higher than normal risk, like in first degree relatives of type 1 diabetic patients, the proinsulin/C-peptide ratio is a complementary risk parameter to HLA genotyping and to the detection of autoantibodies (Dr. Truyen, Belgian Diabetes Registry, unpublished observations). The blood samples of this study were taken at random (i.e., not always fasting), and even when measured this way, the proinsulin/C-peptide ratio provided a dynamic parameter, the screening of which may be simpler and more cost effective than a complete IVGTT or OGTT.

An aim of the present invention is therefore to provide a method for determining with high precision the proinsulin/C-peptide ratio.

The inventors recognized that when the ratio is estimated from concentrations of proinsulin and C-peptide determined in separate reaction compartments, common liquid handling errors (e.g., disparities in the amount of sample added to the reaction compartments), may impose high degree of imprecision on the ratio. The inventors realized that the detrimental effect of such liquid handling errors can be in large eliminated when the concentrations of proinsulin and of C-peptide in a sample are determined within the same reaction compartment. In this case, any variation in the amount of sample or in the amount or quality of reagents added to that compartment affects both measured concentrations to the same extent, and therefore does not affect the proinsulin/C-peptide ratio. Likewise, any variation in incubation and washing steps does not affect the proinsulin/C-peptide ratio. In addition, the history of sample handling (e.g., the number of freeze-thaw cycles, dilution, and additional components) is identical for both determined concentrations. The inventors also realized further advantages of determining the concentrations of proinsulin and of C-peptide in the same reaction compartment, including, for example, reduction in the amount of sample and reagents needed, and reduction in liquid handling, which decreases the likelihood of errors and the workload. Reduction in the amount of sample needed can be particularly advantageous when the sample is derived from a limited source, e.g., from a sample of blood drawn from a small animal, such as from mouse.

The inventors also recognized that the precision of the proinsulin/C-peptide ratio can further be increased when the same capture antibody is used to bind both proinsulin and C-peptide from the sample. This eliminates variations that could otherwise arise due to different binding efficiencies of separate capture antibodies or different quantities of such capture antibodies.

Accordingly, the present invention aims to achieve high precision of the proinsulin/C-peptide ratio by determining the concentrations of analytes relevant for establishing the ratio within the same reaction compartment and using the same capture antibody to bind the analytes from the sample.

The inventors were knowledgeable of the method described by Hemmila et al. (Clin Chem 1987;33: 2281-3). There, lutropin (LH) and follitropin (FSH) were measured in a sample within the same reaction compartment and both proteins were captured from the sample by the same capture antibody, which recognized the alpha chain present in both proteins. Each protein was subsequently detected using an antibody specific for the beta domain which is distinct in each protein. The latter antibodies were labeled with Terbium (anti-beta follitropin) and Europium (anti-beta lutropin) so that the signals could be distinguished by time-resolved fluorescence.

The inventors recognized that the method of Hemmila et al. (1987) could not be adapted for the present aim, because it requires that the two proteins each comprise a distinct specific portion. In contrast, proinsulin comprises the entire sequence of C-peptide and C-peptide does not comprise any portion which is not present in proinsulin. Hence, the inventors faced the problem of how to distinguish C-peptide from proinsulin despite the fact that C-peptide does not comprise any specific sequence not comprised in proinsulin.

The inventors realized that this problem can be solved by determining on one hand the concentration in a sample of proinsulin and on the other hand the concentration of all molecules comprising C-peptide. In physiologically relevant samples (e.g., in serum or plasma), molecules comprising C-peptide will typically contain two predominant forms - proinsulin and C-peptide. Therefore, the concentration of C-peptide in these samples may be determined by subtracting the concentration of proinsulin from the concentration of all molecules comprising C-peptide. The proinsulin/C-peptide ratio can then be readily computed.

Accordingly, in one aspect the present invention provides a method to determine the concentrations in a sample of proinsulin and of molecules comprising C-peptide, wherein the concentrations are determined within the same reaction compartment and using a common capture antibody specific for an epitope within C-peptide, as defined in claim 1.

Proinsulin and molecules comprising C-peptide whose concentrations are to be determined according to the present method may be of either human or animal origin. In the latter case, proinsulin and molecules comprising C-peptide may originate, for example, from mouse, rat, dog, cat or pig, or from another animal species, and in particular such species commonly employed in laboratory practice. Accordingly, the sample in which the concentrations of proinsulin and molecules comprising C-peptide are to be determined may be obtained or derived from either a human or an animal (e.g., an animal as above, i.e., mouse, rat, dog, cat or pig, or another animal species, especially one commonly employed in laboratory practice), or if not directly obtained or derived from a human or an animal, may contain proinsulin and molecules comprising C-peptide with amino acid sequence corresponding to such molecules of human or animal origin.

In another aspect the present invention provides innovative combinations of antibodies suitable as the capture antibody and detection antibodies in the above method, wherein said antibodies bind to their respective epitopes within proinsulin and C-peptide without mutual steric hindrance. Accordingly, the present invention provides a kit-of-parts for use in the above method, comprising such capture antibody and suitable detection antibodies.

The specification further describes that a generalized equivalent of the above method may be useful in any situation in which the concentrations in a sample need to be determined for two or more analytes, all of which comprise a common portion, and each of which, or each of which except one, comprises a distinct specific portion. As a non-limiting example, in the case of proinsulin and C-peptide, the portion common to both analytes would be C-peptide, and the distinct portion specific to proinsulin would be the A and B chains of insulin.

Accordingly, the specification also describes a method to determine the concentrations in a sample of *n* analytes from a group of *N* analytes and the total concentration of the *N* analytes, *N* being 2 or more and *n* being any integer value from 1 to *N*, and each of the *N* analytes comprising at least one common portion present in each other of the *N* analytes, and the *n* analytes comprising each a distinct portion not present in any other of the *N* analytes, wherein the concentrations are determined within the same reaction compartment and using a capture antibody specific for an epitope within the common portion.

Hence, all *N* analytes will be bound by the capture antibody, and the total concentration of the *N* analytes can be determined, as well as the individual concentrations of the *n* analytes. The individual concentrations of the analytes can be used to calculate mutual concentration ratios. Alternatively, a concentration ratio of one or more of the n analytes to the total concentration of the *N* analytes can be determined. This may be useful, e.g., if variations in such ratio has physiological significance.

When individual concentrations are determined for *N-1* analytes, each having a distinct specific portion, the concentration of the *N^{th}* analyte can be easily calculated by subtracting these individual concentrations from the total concentration of the *N* analytes. This may be useful, for example, when the *N^{th}* analyte does not comprise a distinct specific portion or when there is no antibody available for its distinct portion. If the concentration of the *N^{th}* analyte can be (and also is) determined directly, the total concentration of the *N* analytes may be used, for example, as a control for precision of the individual measurements.

Useful applications of the above method include, for example:
- Determining the concentration ratio of proinsulin to insulin. Here, the common portion is represented by the A and B chains of insulin and the distinct portion specific to proinsulin is C-peptide. Concentration of insulin is calculated by subtracting the concentration of proinsulin from the total concentration of molecules comprising insulin.
- Determining the concentration ratios of prohormone(s) versus mature hormone. The common portion is represented by the hormone sequence and the distinct portion(s) specific to prohormone(s) are the parts eliminated during maturation.
- Determining the concentration ratios of different members of protein families, if the member proteins comprise at least one common portion and specific distinct portions. An example may be the group of pituitary hormones (e.g., thyrotropin, chorionic gonadotropin, luthropin and follitropin), each of which comprises a common subunit (alpha) and a specific subunit (beta).
- Determining the concentration ratios of a normal protein and its mutated or modified (e.g., glycosylated or phosphorylated) counterpart. Here, the mutation or modification itself, or the region of protein containing (or not containing) the mutation or modification, will constitute the distinct portion specific to each protein.
- Determining the concentration ratio of different enzyme isoforms from one tissue (e.g., lactate dehydrogenase), or from different tissues (e.g., heart vs. brain creatin kinase, pancreas vs. brain glutamate decarboxylase), if such isoforms comprise at least one common portion and specific distinct portions.

The different molecules whose concentrations and concentration ratios are to be determined as described above may be of either human or animal origin. In the latter case, such molecules may originate, for example, from mouse, rat, dog, cat or pig, or from another animal species, and in particular such species commonly employed in laboratory practice. Accordingly, the sample in which the concentrations of such molecules are to be determined may be obtained or derived from either a human or an animal (e.g., an animal as above), or if not directly obtained or derived from a human or an animal, may contain molecules with amino acid sequence corresponding to such molecules of human or animal origin.

### Detailed Description of the Invention

In one aspect the present invention provides a method to determine the concentrations in a sample of proinsulin on one hand, and of molecules comprising C-peptide on the other hand, wherein the concentrations are determined within the same reaction compartment and using a capture antibody which is specific for an epitope within C-peptide, as defined in claim 1.

The term "concentration" refers both to molar concentration (mol/liter) and to weight concentration (gram/liter).

The term "proinsulin" refers to human proinsulin, as well as to animal proinsulin. Human proinsulin encompasses full-length proinsulin having protein sequence as in GenBank (accession: IPHU, amino acids 25-110, http://www.ncbi.nlm.nih.gov/Genbank/), FVNQHLCGSH LVEALYLVCG ERGFFYTPKT RREAEDLQVG QVELGGGPGA GSLQPLALEG SLQKRGIVEQ CCTSICSLYQ LENYCN, or a similar sequence comprising amino acid substitutions due to normal genetic variation in human populations or due to pathogenic mutations. Usually, normal genetic variation will lead to no more than 10 amino acid substitutions and typically less than 5 amino acid substitutions. Human proinsulin further encompasses proinsulin having any one of disulfide bonds between C19 and C85, between C71 and C76, or between C7 and C72 of the above sequence. Human proinsulin also includes proinsulin having any two or all three such disulfide bonds.

Human proinsulin further encompasses partially processed forms, including: '32-33 split' form resulting from the cleavage of the peptide bond between R32 and E33, '65-66 split' form resulting from the cleavage of the peptide bond between R65 and G66, 'des 31, 32' form resulting from elimination of R31 and R32 from the '32-33 split' form, 'des 64, 65' form resulting from elimination of K64 and R65 from the '65-66 split' form, as well as any intermediate forms arising in the course of this processing. In the above forms, the protein chains are connected by at least one of the disulfide bonds C19-C85 or C7-C72, and typically all three disulfide bonds are present. Human proinsulin may also encompass truncation products of the above partially processed forms which may result from elimination of one or more amino acids from the free termini of the partially processed forms or from internal cleavage within the partially processed forms, for example, by action of non-specific proteases.

Human proinsulin further encompasses any modified (e.g., glycosylated, phosphorylated, or labeled) forms of full-length, partially processed or further truncated proinsulin.

Animal proinsulin encompasses, for example, proinsulin from mouse, rat, dog, cat or pig, or from another animal species, and in particular such species commonly employed in laboratory practice. For example, pig proinsulin may have protein sequence as in GenBank (accession: P01315, amino acids 25-108), FVNQHLCGSH LVEALYLVCG ERGFFYTPKA RREAENPQAG AVELGGGLGG LQALALEGPP QKRGIVEQCC TSICSLYQLE NYCN. For example, dog proinsulin may have protein sequence as in GenBank (accession: P01321, amino acids 25-110), FVNQHLCGSH LVEALYLVCG ERGFFYTPKA RREVEDLQVR DVELAGAPGE GGLQPLALEG ALQKRGIVEQ CCTSICSLYQ LENYCN. For example, two different organ-specific proinsulin forms are present in rat - proinsulin 1 may have protein sequence as in GenBank (accession: P01322, amino acids 25-110), FVKQHLCGPH LVEALYLVCG ERGFFYTPKS RREVEDPQVP QLELGGGPEA GDLQTLALEV ARQKRGIVDQ CCTSICSLYQ LENYCN; proinsulin 2 may have protein sequence as in GenBank (accession: P01323, amino acids 25-110), FVKQHLCGSH LVEALYLVCG ERGFFYTPMS RREVEDPQVA QLELGGGPGA GDLQTLALEV ARQKRGIVDQ CCTSICSLYQ LENYCN.

A skilled person will readily appreciate that the considerations as detailed for human proinsulin regarding the presence of amino acid substitutions due to normal genetic variation or pathogenic mutations, the presence of disulfide bonds, the presence of partially or completely processed forms, further truncated forms and modified forms will analogously apply to animal proinsulin.

The term "C-peptide" refers to a peptide chain (amino acids 33 to 63 of the above human proinsulin sequence or the corresponding part of an animal proinsulin sequence) which connect the A chain of insulin (amino acids 66 to 86 of the above human proinsulin sequence or the corresponding part of an animal proinsulin sequence) with the B chain of insulin (amino acids 1 to 30 of the above human proinsulin sequence or the corresponding part of an animal proinsulin sequence) in a proinsulin molecule. C-peptide is comprised in full-length proinsulin, as well as in the '32-33 split', '65-66 split', 'des 31, 32', and 'des 64, 65' forms of proinsulin, and in potential truncation products thereof. Because C-peptide forms a portion of proinsulin, the above considerations regarding the human or animal origin of and the potential amino acid substitutions within proinsulin may be extended to C-peptide. C-peptide is released from human proinsulin by cleaving the latter at both R32-E33 bond and R65-G66 bond or from animal proinsulin by cleavage at corresponding sites. Such 'free' C-peptide encompasses peptides E33 to R65, E33 to K64, and E33 to Q63 or the corresponding peptides in case of animal C-peptide, as well as any truncation products thereof which may result from elimination of one or more amino acids from the free termini of C-peptide or from its internal cleavage, for example, by action of non-specific proteases. C-peptide further encompasses any modified (e.g., glycosylated, phosphorylated, or labeled) forms of C-peptide.

When this specification refers to proinsulin, C-peptide or molecules comprising C-peptide, it must be understood that such molecules may be of human or animal origin. Where particular sequence or amino acid positions within proinsulin or C-peptide are specified, this mostly refers to human proinsulin or C-peptide, and in particular to the human proinsulin sequence as listed above, but it must be understood that a skilled person can readily extend these considerations to proinsulin and C-peptide of any animal species.

Accordingly, in an embodiment, proinsulin and molecules comprising C-peptide are of human origin. In another embodiment, proinsulin and molecules comprising C-peptide are of animal origin. In one useful embodiment, proinsulin and molecules comprising C-peptide originate from mouse, rat, dog, cat or pig.

When the present specification refers to the "concentration of C-peptide", this means the concentration of 'free' C-peptide, i.e., C-peptide released from the proinsulin molecule as detailed above. Accordingly, when the present specification refers to the ratio of the concentration of proinsulin to the concentration of C-peptide (proinsulin/C-peptide ratio), the latter means free C-peptide.

The term "molecules comprising C-peptide" refers to all molecules comprising the C-peptide forms E33 to R65, E33 to K64, or E33 to Q63, as well as molecules comprising any further truncated forms of C-peptide if this includes at least 15, preferably at least 20, more preferably at least 25 and most preferably at least 28 consecutive amino acids of C-peptide.

The molecule is typically a polypeptide or a modified polypeptide, such as glycosylated, phosphorylated or labeled polypeptide, or a polypeptide comprising disulfide bonds. Apart from the C-peptide portion, such molecule will usually comprise more than 5 additional amino acids. In the present context, molecules comprising C-peptide will primarily include C-peptide in any of its forms, and mainly in its E33 to R65, E33 to K64, and E33 to Q63 C forms, and proinsulin in any of its forms, and mainly in its full-length, '32-33 split', '65-66 split', 'des 31, 32', and 'des 64, 65' forms. These forms are likely to be most relevant in the physiological situation and thus to be present in a sample comprising, for example, blood serum or plasma.

The method of the present invention allows for determining the concentrations in a sample of proinsulin and of molecules comprising C-peptide. Because molecules comprising C-peptide in a physiologically relevant sample primarily include C-peptide and proinsulin, the difference between the concentration of these molecules and the concentration of proinsulin may be considered an estimate of the concentration of C-peptide (i.e., free Peptide). From these values the proinsulin/C-peptide ratio can be constructed.

The method of the present invention allows for determining the above concentrations within the same reaction compartment. The term "reaction compartment" refers to an enclosed space delineated from the outside environment or from neighboring reaction compartments by physical barriers (e.g., walls) which do not allow passage of reaction components. A reaction compartment is capable of holding a reaction mixture and the material forming the walls of the reaction compartment is chemically inert to components of the reaction mixture and physically inert to conditions used in the present method. The inner walls of the reaction compartment may allow or may be adapted to allow for covalent or non-covalent attachment of a capture antibody. Reaction compartments useful in the present invention comprise individual wells of microtiter plates of various formats and reaction tubes of various volumes. Microtiter plates are particularly useful in the present invention.

The present method determines the concentrations of proinsulin and of molecules comprising C-peptide "within the same reaction compartment", i.e., the concentration of both components in a sample placed into one reaction compartment is determined by carrying out all required reaction and measurement steps within this reaction compartment.

The term "sample" refers to any composition that can be analyzed using the method of the present invention. A sample is generally an aqueous composition. A useful sample comprises a relevant biological material, such as body fluid (e.g., blood, serum, plasma, saliva, sweat, or urine of a human or animal subject) of a human or animal subject, homogenates prepared from tissues of a human or animal subject, and lysates and supernatants of cultured cells or cell lines, especially of eukaryotic cells or cell lines. Such biological material may preferably originate from a human subject and may more preferably comprise blood, serum or plasma derived from a human subject. Such biological material may also originate from an animal, such as, for example, mouse, rat, dog, cat or pig, or another animal species, especially one commonly employed in laboratory practice, and may more preferably comprise blood, serum or plasma derived from this animal. The sample may further comprise other components, for example, water to dilute the biological material, and components to maintain pH of the sample in a particular range (e.g., between 6.0 and 8.5, and preferably between 7.0 and 8.0); to control ionic strength of the sample (e.g., salts); to prevent degradation of proteins in the sample (e.g., protease inhibitors and EDTA); to stabilize protein disulfide bonds (e.g., reducing agents); to prevent non-specific interactions (e.g., blocking agents); and other components. Such components are commonly included in samples for separate immunoassay analysis of proinsulin and C-peptide and are known to a skilled person.

The term "antibody" refers to a protein or glycoprotein produced in response to an antigen, being specific for an epitope within the antigen, and binding to the epitope via non-covalent interactions. Herein, "antibody" refers to polyclonal and monoclonal antibodies, as well as to single-chain antibodies, hybrid antibodies, chimeric antibodies, and any protein (e.g., a recombinant protein) comprising the antigen-binding function of a parent antibody. The term "antibody" encompasses whole immunoglobulins and any fragments thereof which retain the antigen-binding function of a parent antibody, e.g., Fab, F(ab')2, and Fv fragments. The term "antibody" further encompasses both the actual immunoglobulin molecules or a fragment thereof, as well as a population of such molecules, and a preparation comprising such molecules. A preparation of polyclonal antibody may include, for example, an immune serum from an organism immunized against an antigen of interest or a preparation of polyclonal antibodies affinity-purified from such serum. A preparation of monoclonal antibody may include, for example, ascitic fluid, supernatant of cultured hybridoma cells, or a preparation of purified monoclonal antibodies in a suitable buffer.

The term "polyclonal antibody" refers to a heterogeneous antibody population. This may comprise antibody molecules having antigen-binding functions specific for distinct epitopes within the same antigen. Polyclonal antibodies of any mammalian species can be used in the present invention. Useful examples are polyclonal antibodies from rabbit, goat, donkey and sheep. Methods of preparing polyclonal antibodies are known in the art, as are methods for their affinity purification.

The term "monoclonal antibody" refers to a homogeneous antibody population. The term is not limited regarding the species, and monoclonal antibodies of any mammalian species can be used in the present invention. Monoclonal antibodies of murine or rat origin are especially useful because of the availability of murine and rat cell lines for use in preparing of hybridomas to produce monoclonal antibodies. Methods of preparing monoclonal antibodies are known in the art, as are methods of determining their specificity.

The specificity and efficiency of binding an epitope within an antigen may be more reproducible for a monoclonal antibody preparation than for a polyclonal antiserum, because monoclonal antibodies are currently obtained from a continuous source (e.g., cultured hybridomas), and different preparations of monoclonal antibodies obtained from such source have essentially identical properties. On the contrary, polyclonal antibodies are typically prepared from a limited source, such as from a particular bleed of an immune serum and properties of polyclonal antibodies may differ between different bleeds. For these and other reasons, the use of monoclonal antibodies may be preferred in the method of the present invention. Accordingly, in an embodiment, at least one of the capture and detection antibodies is a monoclonal antibody. In another embodiment, the capture antibody and each detection antibody are monoclonal antibodies.

The term "epitope" refers to a binding site for an antibody on an antigen, which herein is usually a polypeptide molecule. An antibody "specifically binds to" or is "specific for" an epitope within a molecule, such as a polypeptide molecule, when it binds to that particular epitope without substantially binding to any other epitope on the same or another molecule. Accordingly, when an antibody which is specific for a certain epitope within a molecule, such as a polypeptide molecule, is said to "bind to the molecule", this means that the antibody specifically binds to the epitope within the molecule. The dissociation constant (K_{d}) of such specific binding is typically 10⁻⁷ to 10⁻¹¹ M. An epitope may be formed by a consecutive sequence of amino acids, typically between 5 and 15 consecutive amino acids. An epitope may also be formed by amino acids, some or all of which are not in a consecutive sequence, but which contribute to a particular three-dimensional structure within a protein. An epitope may also be formed as a consequence of a specific modification to a protein (e.g., glycosylation or phosphorylation). Some epitopes may only be present when a protein assumes its native conformation. Other epitopes may be present in both folded and denatured proteins. Yet other epitopes may only be present in denatured proteins. Typically, the present method does not denature proteins prior to their binding to an antibody. Therefore, antibodies capable of recognizing epitopes in non-denatured proteins may be preferred. An epitope as defined by a specific sequence of amino acids will also include epitopes having amino acid substitutions, if such epitopes are bound by the corresponding antibody in an equivalent manner or extent.

The following further applies to the present specification. If an epitope is said to be present within a polypeptide, then the polypeptide molecule comprises the epitope. If an epitope is said not to be present within a polypeptide, then the polypeptide molecule does not comprise the epitope even while it may comprise a portion of the amino acid sequence that forms the epitope. If an epitope is said to be present within a polypeptide but not within a specific portion of that polypeptide, then this portion may comprise amino acids which do contribute to the epitope, but the portion on its own or when placed into a different polypeptide context, does not comprise the epitope. If an epitope is said not to comprise any sequence from a specific portion of a polypeptide, then no one amino acid from this portion contributes to the epitope.

The method of the present invention provides a capture antibody specific for an epitope within C-peptide. "Epitope within C-peptide" means an epitope entirely contained within C-peptide, i.e., contributed to only by amino acids from C-peptide. Such epitope will be present in C-peptide (i.e., 'free' C-peptide), as well as in essentially all molecules comprising C-peptide, including proinsulin. Consequently, the capture antibody can specifically recognize and bind to molecules comprising C-peptide from the sample, including C-peptide and proinsulin. Accordingly, the present method uses a common capture antibody to bind both C-peptide and proinsulin from the sample, rather than using a distinct capture antibody for each of these molecules. The present method may preferably use one such common capture antibody.

The capture antibody should further allow for separating the molecules or complexes of molecules (e.g., C-peptide or proinsulin specifically bound by other antibodies) which are specifically bound by the capture antibody from other components of the sample or from other reagents employed in the method. For example, the capture antibody may typically be immobilized on a solid support. Here, the capture antibody and molecules or complexes of molecules specifically bound by it are retained on the solid support, while other components of the sample or other reagents may be easily removed by washing steps.

Accordingly, in an embodiment of the present invention the capture antibody is immobilized on a solid support. Solid support encompasses a continuous solid phase, as well as a discontinuous solid phase of discrete particles, such as agarose beads or magnetic beads. For example, micro-carriers like spheres or beads suspended in the reaction mixture may be used as solid support to immobilize the capture antibody. Solid support of the present invention allows for immobilization of the capture antibody thereon, and is compatible with any incubation, washing or detection steps of the present methods. Where a detection step involves measuring of an optical output (e.g., color, fluorescence, luminescence), the solid support may not interfere with these measurements, or the interference caused by the solid support must be minimal and constant so that it can be subtracted as a blanc value from all measurements. Other than that, solid support in the present invention may be any material commonly used in immunoassay methods or multiplex analysis. A preferable solid support of the hereby presented method may be a microtiter plate.

The term "immobilized antibody" refers to an antibody linked on a solid support. Immobilization of the capture antibody facilitates the recovery of the antibody and any proteins bound to the antibody following any incubation, washing and detection steps of the method. Immobilization of the capture antibody on a solid support may occur via covalent bonds or non-covalent binding which is sufficiently stable to endure any incubation, washing and detection steps of the method of the invention. An immobilized capture antibody is present on the surface of a solid support and is oriented so that its antigen-binding portion is available for contacting the molecules present in the reaction mixture. If solid support is a microtiter plate, the capture antibody is preferably immobilized within wells of the microtiter plate, so that the antigen-binding portion of the antibody faces the interior space of the wells, which form the reaction compartments. Immobilization of capture antibodies on solid support is routine in immunoassay methods and any common protocols for doing so may be employed in the present invention. As an example, a capture antibody may be immobilized by allowing it to form non-covalent bonds with hydrophobic solid support, such as polystyrene. Remaining sites on the solid support which have the capacity to non-specifically bind proteins are blocked with a blocking agent, such as BSA, before the solid support is contacted with a sample.

In an embodiment of the present invention, the capture antibody is immobilized on a solid support prior to incubating the antibody with the sample. In another embodiment, the capture antibody may first be incubated with the sample and only subsequently immobilized on a solid support.

The method of the present invention to determine the concentrations in a sample of proinsulin and of molecules comprising C-peptide, comprises:
(i.) binding of the capture antibody to molecules comprising C-peptide from the sample,
(ii.) binding of a detection antibody specific for an epitope within C-peptide, said epitope being different from the epitope for which the capture antibody is specific, to molecules comprising C-peptide from the sample, and
(iii.) binding of another detection antibody specific for an epitope within proinsulin, said epitope not being present in C-peptide, to proinsulin from the sample,
wherein the bindings take place within the same reaction compartment.

The method thus includes providing a capture antibody specific for an epitope within C-peptide, as detailed above, and allowing this capture antibody to bind to those molecules from the sample, which comprise C-peptide.

The method further includes providing a detection antibody specific for an epitope within C-peptide, said epitope being different from the epitope for which the capture antibody is specific, and allowing this detection antibody to bind to those molecules from the sample, which comprise C-peptide.

In an embodiment the epitope within C-peptide which is recognized by this detection antibody is not contributed to by any amino acids which contribute to the epitope recognized by the capture antibody, and no steric hindrance in binding to C-peptide occurs between the two antibodies. For example, when each epitope is formed by a consecutive sequence of amino acids, the sequences do not overlap. Preferably, such sequences may be separated by at least 5, and more preferably at least 10 amino acids which do not contribute to either epitope. For example, the capture antibody may be specific for an epitope within the N-terminal half of C-peptide and the detection antibody may be specific for an epitope in the C-terminal half of C-peptide, or vice versa.

In an embodiment the epitopes and antibodies are chosen such that binding of the capture antibody to its epitope within proinsulin or to another molecule comprising C-peptide does not prevent or decrease the efficiency of binding of the detection antibody specific for an epitope within C-peptide to the same molecule, and binding of the detection antibody specific for an epitope within C-peptide to proinsulin or to another molecule comprising C-peptide does not prevent or decrease the efficiency of binding of the capture antibody to its epitope within the same molecule. Useful examples of suitable combinations of antibodies may include as a capture antibody PEP-001 (proposed epitope: GSLQK, amino acids 60 to 64 of the above human proinsulin sequence) (Kjems LL et al Clin Chem 1993; 39:2146-50) and as detection antibody specific for an epitope in C-peptide CPT3F11 or CPTF3F8 (proposed epitope: DLQVGQV, amino acids 36 to 42 of the above human proinsulin sequence) (NOVO Nordisk). The above antibodies are specific for epitopes in human C-peptide and therefore are most suited to bind proinsulin and molecules comprising C-peptide of human origin. A skilled person would appreciate that these antibodies may not always perform optimally in binding proinsulin and molecules comprising C-peptide of animal origin, and that efficient binding of such molecules of animal origin may require antibodies specifically raised for such molecules.

The method further includes providing another detection antibody specific for an epitope within proinsulin, said epitope not being present in C-peptide, and allowing this detection antibody to bind to proinsulin molecules from the sample. A useful example of such detection antibody may include HUI-001 (proposed epitope: FYTP, amino acids 25 to 28 of the above human proinsulin sequence) (NOVO Nordisk). The above antibodies are specific for epitopes in human proinsulin and therefore are most suited to bind proinsulin of human origin. A skilled person would appreciate that these antibodies may not always perform optimally in binding proinsulin of animal origin, and that efficient binding of such proinsulin may require antibodies specifically raised for such proinsulin.

In an embodiment the epitope within proinsulin which is recognized by this antibody is not contributed to by any amino acids from the C-peptide portion of proinsulin. Hence, the epitope is contributed to only by amino acids from the A and/or B chains of insulin.

In an embodiment the epitope and the antibody is chosen such that binding of the antibody to its epitope within a proinsulin molecule is not prevented or decreased in efficiency by binding of the capture antibody and/or binding of the detection antibody specific for an epitope within C-peptide to the same molecule, and that binding of the antibody to its epitope within a proinsulin molecule does not prevent or decrease the efficiency of binding of the capture antibody and/or of the detection antibody specific for an epitope within C-peptide to the same molecule.

Hence, in an embodiment, binding of any one of the capture or detection antibodies to its respective epitope within proinsulin or a molecule comprising C-peptide does not prevent or decrease the efficiency of binding of any other of the capture or detection antibodies to its respective epitope within the same molecule.

In another embodiment, cumulative binding of any two of the capture or detection antibodies to their respective epitopes within proinsulin does not prevent or decrease the efficiency of binding of the third antibody to its respective epitope within the same molecule.

In an embodiment, the method of the present invention comprises one or more incubation steps which allow for the binding of the capture antibody and of the detection antibodies to their respective epitopes within proinsulin and molecules comprising C-peptide from the sample.

The antibodies may be allowed to bind to their epitopes (i.e., allowed to become bound to their epitopes) in (i.e., during) separate incubation steps. Accordingly, in an embodiment, each of the capture antibody and the detection antibodies is allowed to bind to its respective epitopes in a distinct incubation step.

In another embodiment, any two of the capture antibody and the detection antibodies are allowed to bind to their respective epitopes in one incubation step (i.e., bind simultaneously), while the remaining antibody would be allowed to bind its epitope in a distinct incubation step.

The antibodies may also be all allowed to bind to their epitopes in one incubation step. Accordingly, in an embodiment, the capture antibody and the detection antibodies are allowed to bind to their respective epitopes in one incubation step.

Incubation steps in which more than one antibody is allowed to bind to its epitope may be preferred due to shortening the overall duration of the assay.

An "incubation step" comprises both contacting an antibody with a sample or components thereof and incubating these together to allow for specific binding to take place. Conditions of incubation (e.g., temperature, duration, presence of blocking agents, composition of the reaction mixture, etc.) are chosen such as to obtain maximal binding of an antibody to its epitope within a particular protein. These conditions are generally known to a skilled person and can be readily optimized by a skilled person.

The goal of the present method is to be quantitative. Hence, in measuring the concentrations of proinsulin and molecules comprising C-peptide, the method should ideally detect the presence of each and every such molecule in the sample. This would require that ideally each and every proinsulin molecule from the sample is bound by the capture antibody and by each detection antibody, and each and every molecule comprising C-peptide from the sample is bound by the capture antibody and by at least the detection antibody specific for an epitope within C-peptide.

In an embodiment, after the binding of the capture antibody and of the detection antibodies to their respective epitopes within proinsulin and molecules comprising C-peptide from the sample, essentially all proinsulin (i.e., essentially all proinsulin molecules) from the sample is bound by the capture antibody and by each detection antibody, and essentially all molecules comprising C-peptide from the sample are bound by the capture antibody and by at least the detection antibody specific for an epitope within C-peptide. Herein, "essentially all" refers to 80% or more, preferably 90% or more, more preferably 95% or more and most preferably 99% or more of the molecules.

To achieve such binding of "essentially all" available epitopes by the corresponding antibodies requires that the antibodies are provided in molar excess to the antigen present (e.g., molar ratio of >1:10, or >1:100, or >1:1000 of antigen to antibody) and that incubation conditions are optimal. Determining the required amount of antibodies and optimizing the conditions of incubation are all within the abilities of a skilled person.

In an embodiment, the method comprises one or more washing steps to remove components of the sample which are not bound to the capture antibody or to remove detection antibodies which are not bound to their respective epitopes in proinsulin and in molecules comprising C-peptide from the sample.

Washing steps are included to decrease background and ensure the specificity of the signal eventually obtained during detection. Hence, a signal should ideally only be obtained from detection antibodies which are specifically bound to their respective epitopes in proinsulin and molecules comprising C-peptide, which in turn are specifically bound to the capture antibody. Components of the sample which are removed in washing steps include components which remain unbound (i.e., in solution), as well as components which bind non-specifically to solid support or to the capture antibody. Conditions and compositions used in washing should be optimized to maximally disrupt non-specific interactions, without disrupting specific antibody-antigen interactions. These conditions and compositions are known to a skilled person and can be readily optimized by a skilled person. One or more washing steps may be included after any or all incubation steps, or only after the last incubation step.

In an embodiment, the present method comprises one or more steps which allow for detecting the presence of detection antibodies (ideally all of which are specifically bound to their respective epitopes within proinsulin and molecules comprising C-peptide) in the reaction compartment. These "detection steps" are normally included after the incubation and washing steps.

In an embodiment, each of the detection antibodies comprises a different detectable element. Hence, the detection antibody specific for an epitope within C-peptide will contain a different detectable element than the detection antibody specific for an epitope within proinsulin which is not present in C-peptide. This facilitates detection of the antibodies and distinguishing the signals coming from the different detection antibodies.

Accordingly, in an embodiment, the method comprises one or more steps which allow for detecting the detectable elements comprised in the detection antibodies, such that signals obtained from the different detectable elements can be distinguished.

The term "detectable element" refers to any element present within or added to a detection antibody that can be detected so as to produce a measurable signal. A detectable element may directly produce a readable signal, such as a radioactive isotope or a fluorescent moiety (when illuminated with light of appropriate wavelength).

Detection of a detectable element may require further reaction steps to generate a measurable signal. For example, a detectable element may be an enzyme linked to the antibody, which when provided with suitable reactants generates light (chemiluminescence) or a colored reaction product (colorimetry).

In a preferred embodiment, a detectable element may be a lanthanide atom linked to the antibody, which may be subsequently dissociated from the antibody in a specific solution and made to form fluorescent chelates, which may be measured. The use of lanthanide atoms may be preferred in the present method, because when chelated, different lanthanide atoms (e.g., Terbium, Europium or Samarium) produce time-resolved fluorescence signals which can be differentiated on the basis of different wavelengths of the emitted light and different emission kinetics. Accordingly, in an embodiment, each detection antibody is labeled by a different lanthanide atom label and preferably one chosen from Terbium, Europium or Samarium. The detection antibody recognizing the component which is less prevalent in the sample (here typically proinsulin) may preferably be labeled by Europium, which produces the strongest signal.

A detectable element may require further recognition by a molecule with high affinity to this element, said molecule in turn comprising a detectable element, such as, for example, one of the above. For example, an antibody may comprise one or more biotin molecules, which may subsequently be recognized by streptavidin, which comprises a detectable element. A detectable element may also be the Fc portion of the detection antibody. This will be different between the antibodies if they are derived from different organisms and thus may be distinguished.

A detection antibody may comprise one detectable element or more identical detectable elements, the latter resulting in amplification of the signal. Such signal amplification may be preferred when one of the measured proteins is present at considerably lower levels than the other one (e.g., proinsulin typically comprises about 1 to 5% of molecules comprising C-peptide and thus amplification may be desirable; an example of amplifying the proinsulin signal is shown in Example 1).

These and other detectable elements are commonly used in multi-analyte immunoassays and their use and methods of their detection are well known to a skilled person.

Any combination of detectable elements may be used in the present invention, provided it allows for generation of signals that can be distinguished and that do not interfere with each other (i.e. do not influence each other's detection). The detectable elements may be detected simultaneously or subsequently to each other.

In an embodiment, the method comprises one or more steps which allow for quantitative measuring of the signals and comparing the measured signal intensities (i.e., signa) intensities as obtained by the quantitative measuring) to references, whereby the concentrations in the sample of proinsulin and of molecules comprising C-peptide are determined.

Methods and protocols for measuring of the signals obtained in the detection steps depend on the nature of the detectable element and are known in the art. Similarly methods for making calibrator series (references) by detecting known concentrations of analytes and estimating concentration of the analytes in a sample by comparing the measured signal intensities to calibration curves are well known in the art.

In another aspect the present invention provides a kit of parts for use in the method of the present invention to determine the concentrations in a sample of proinsulin and of molecules comprising C-peptide, comprising:
(i.) a capture antibody specific for an epitope within C-peptide,
(ii.) a detection antibody specific for an epitope within proinsulin, said epitope not being present in C-peptide, and
(iii.) another detection antibody specific for an epitope within C-peptide, said epitope being different from the epitope for which the capture antibody is specific.

In embodiment the kit comprises antibodies specific for proinsulin and molecules comprising C-peptide of human origin. In an embodiment the kit comprises the antibodies PEP-001, CPT3F11 and HUI-001. in another embodiment the kit comprises the antibodies PEP-001, CPT3F8 and HUI-001. In an embodiment, PEP-001 is used as a capture antibody and CPT3F11 and HUI-001 are used as detection antibodies. In another embodiment, PEP-001 is used as a capture antibody and CPT3F8 and HUI-001 are used as detection antibodies. In an embodiment, CPT3F11 or CPT3F8 is used as a capture antibody and PEP-001 and HUI-001 are used as detection antibodies.

In an embodiment the kit comprises antibodies specific for proinsulin and molecules comprising C-peptide of animal origin. In an embodiment such animal is mouse, rat, dog, cat or pig, or another animal species, and in particular such species commonly employed in laboratory practice.

In an embodiment, each of the detection antibodies comprised in the kit comprises a different detectable element. In an embodiment such detectable elements are different lanthanide atoms. In an embodiment, the lanthanide atoms are chosen from Europium, Terbium and Samarium.

In an embodiment, the capture antibody comprised in the kit is immobilized on a solid support.

In an embodiment, the capture antibody comprised in the kit is immobilized within wells of a provided microtiter plate.

In an embodiment, the capture antibody and the detection antibodies comprised in the kit are chosen such that binding of any one of the capture or detection antibodies to its respective epitope within proinsulin or a molecule comprising C-peptide does not prevent or decrease the efficiency of binding of any other of the capture or detection antibodies to its respective epitope within the same molecule.

In an embodiment, at least one of the capture and detection antibodies comprised in the kit is a monoclonal antibody.

In an embodiment, the capture antibody and each of the detection antibodies comprised in the kit are monoclonal antibodies.

In an embodiment, each of the detection antibodies comprised in the kit is contained in separate container.

In an embodiment, both detection antibodies comprised in the kit are contained within the same container.

In an embodiment, both detection antibodies comprised in the kit are contained within the same container and within the same mixture, such as a liquid mixture or a mixture of lyophilized preparations.

In an embodiment, any or all of the capture antibody and the detection antibodies comprised in the kit are provided in solution.

In an embodiment, any or all of the capture antibody and the detection antibodies comprised in the kit are provided as lyophilized preparation.

In an embodiment, the kit comprises instructions to the user for using the kit according to the method of the invention.

The specification further describes a method to determine the concentrations in a sample of *n* analytes from a group of *N* analytes and the total concentration of the *N* analytes, *N* being 2 or more and *n* being any integer value from 1 to *N*, and each of the *N* analytes comprising at least one common portion present in each other of the *N* analytes, and the *n* analytes comprising each a distinct portion not present in any other of the *N* analytes, wherein the concentrations are determined within the same reaction compartment and using a capture antibody specific for an epitope within the common portion.

The method to determine the concentration of proinsulin and molecules comprising C-peptide represents a specific case of the above more general method, namely the case where *N*=2 and *n*=1, wherein the two analytes are proinsulin and C-peptide, wherein the common portion is C-peptide and the specific portion only present in proinsulin are the A and B chains of insulin.

The meaning of terms in the above method, such as the terms "concentration", "reaction compartment", "within the same reaction compartment", "sample", "antibody", "epitope", "antibody specific for an epitope", "binding", "specific binding", "immobilized antibody", "solid support", "incubation step", "washing step", "detectable element", "detection step", "essentially all", and other terms is the same (or equivalent) as described elsewhere in this specification. Similarly, many considerations described elsewhere in this specification regarding incubation, washing, detection and measurement steps may be extended to this method.

In an embodiment, the analyte is a peptide, oligopeptide, polypeptide, protein or a modified form thereof, such as, e.g., a glycosylated, phosphorylated or disulfide bond-containing form. Such molecules may be of human or animal origin.

In an embodiment, an analyte is formed by a single polypeptide chain. In another embodiment, an analyte includes more than one polypeptide chains, wherein said polypeptide chains are linked by one or more covalent bonds, e.g., a disulfide bond.

The term "common portion" refers to a structural feature which is found in each of the *N* analytes. When an analyte is an oligo- or poly-peptide or a protein, such common portion may typically be an amino acid sequence present in all *N* analytes. Such common amino acid sequence may be identical in all *N* analytes or may comprise amino acid substitutions in some analytes. Typically, such common amino acid sequence may show at least 70% homology and preferably at least 80% homology between the analytes. Typically, such common amino acid sequence may comprise at least 5, preferably at least 15, and more preferably at least 20 amino acids. A common portion or common amino acid sequence according to the present invention will comprise at least two distinct epitopes which can be bound by two different antibodies without mutual steric hindrance.

The terms "distinct portion" or "distinct specific portion" refer to a structural feature which is found in one of the *n* analytes, but not in any other of the *N* analytes. When an analyte is an oligo- or poly-peptide or a protein, such distinct portion may be an amino acid sequence. Typically, such distinct amino acid sequence may comprise 1 or more amino acids. For example, such distinct amino acid sequence may comprise 1, 2, 3, 4, 5, more than 5, or more than 10, 20, 30, 40 or 50 amino acids. Alternatively, when an analyte is an oligo- or poly-peptide or a protein, such distinct portion may be a specific modification of one or more of its amino acids, such as glycosylation or phosphorylation. A distinct portion or distinct amino acid sequence present in one of the *n* analytes will comprise or will contribute to at least one epitope which is not found in any other of the *N* analytes.

The above method comprises:
(i.) binding of the capture antibody to analytes from the sample, which comprise the common portion,
(ii.) binding of a detection antibody specific for an epitope within the common portion, said epitope being different from the epitope for which the capture antibody is specific, to analytes from the sample, which comprise the common portion, and
(iii.) binding of *n* detection antibodies, each specific for an epitope within the distinct portion of another of the *n* analytes, to their respective epitopes within the *n* analytes from the sample,
wherein the binding takes place within the same reaction compartment.

The method thus includes providing a capture antibody specific for an epitope within the common portion of the *N* analytes and allowing this capture antibody to bind to those molecules from the sample, which comprise the common portion.

The method further includes providing a detection antibody specific for an epitope within the common portion of the *N* analytes, said epitope being different from the epitope for which the capture antibody is specific, and allowing this detection antibody to bind to those molecules from the sample, which comprise the common portion.

In an embodiment the epitope within the common portion which is recognized by this antibody is not contributed to by any amino acids which contribute to the epitope recognized by the capture antibody. For example, when each epitope is formed by a consecutive sequence of amino acids, the sequences do not-overlap. Preferably, such-sequences may be separated by at least 5, and more preferably at least 10 amino acids which do not contribute to either epitope. For example, the capture antibody may be specific for an epitope within the N-terminal half of the common portion and the detection antibody may be specific for an epitope in the C-terminal half of the common portion, or vice versa.

In an embodiment the epitopes and antibodies are chosen such that binding of the capture antibody to its epitope within an analyte comprising the common portion does not prevent on decrease the efficiency of binding of the detection antibody specific for an epitope within the common portion to the same molecule, and binding of the detection antibody specific for an epitope within the common portion to an analyte comprising the common portion does not prevent or decrease the efficiency of binding of the capture antibody to its epitope within the same molecule.

The method further includes providing *n* detection antibodies, each specific for an epitope within the distinct portion of another of the *n* analytes, and allowing these detection antibodies to bind to the respective analytes from the sample.

In an embodiment the epitopes and the *n* detection antibodies are chosen such that binding of any of the *n* antibodies to its epitope in the respective analyte molecule is not prevented or decreased in efficiency by binding of the capture antibody and/or binding of the detection antibody specific for an epitope within the common portion to the same molecule, and that binding of any of the *n* antibodies to its epitope in the respective analyte molecule does not prevent or decrease the efficiency of binding of the capture antibody and/or binding of the detection antibody specific for an epitope within the common portion to the same molecule.

Hence, in an embodiment, binding of any one of the capture antibody or detection antibodies to its respective epitope within any of the *N* analytes does not prevent or decrease the efficiency of binding of any other of the detection antibodies or the capture antibody to its respective epitope within the same molecule. In another embodiment, cumulative binding of any two of the capture antibody or detection antibodies to their respective epitopes within any analyte does not prevent or decrease the efficiency of binding of the third antibody to its respective epitope within the same molecule.

In an embodiment, the method comprises one or more incubation steps allowing for binding of the capture antibody and of the detection antibodies to their respective epitopes within the analytes. The antibodies may be allowed to bind to their epitopes (i.e., allowed to become bound to their epitopes) in separate or in common incubation steps. The nature of incubation steps is discussed elsewhere in this specification.

In an embodiment, the method comprises one or more washing steps to remove components of the sample which are not bound to the capture antibody or to remove detection antibodies which are not bound to their respective epitopes within the analytes. These sample components and unbound detection antibodies may be removed in separate or in common washing steps. The nature of washing steps is discussed elsewhere in this specification.

In an embodiment, after binding of the capture antibody and of the detection antibodies to their respective epitopes within the analytes, essentially all of the *N* analytes (i.e., essentially all molecules of the *N* analytes) from the sample, are bound by the capture antibody and by at least the detection antibody specific for an epitope within the common portion, and essentially all of the *n* analytes (i.e., essentially all molecules of each of the *n* analytes) are bound by the capture antibody, the detection antibody specific for an epitope within the common portion, and by the detection antibody specific for the distinct portion of each of the *n* analytes. The importance of this consideration for the quantitative nature of the method and the meaning of "essentially all" is described elsewhere in this specification.

In an embodiment, *N* is 2, i.e., the concentration of two analytes is determined.

In an embodiment, *N* is 3 or more, i.e., the concentration of at least one of the analytes and the total concentration of all three or more analytes is determined.

In an embodiment, *N* is 2, 3, 4 or 5.

in an embodiment, *n* is *N*-*1*, i.e., the individual concentrations of each but one of the *N* analytes are determined and the total concentration of all *N* analytes is determined. The concentration of the *N^{th}* analyte may be calculated by subtracting the individual concentrations from the total.

In an embodiment, *n* equals *N*, i.e., the individual concentrations of each of the *N* analytes are determined and the total concentration of all *N* analytes is determined. The total concentration may be used to control the accuracy of the individual measurements, as the sum of individual concentrations must equal the total concentration of all *N* analytes.

In an embodiment, each of the detection antibodies comprises a different detectable element. The nature of such detectable elements is discussed elsewhere in this specification.

In an embodiment, the method comprises one or more steps which allow for detecting the detectable elements comprised in the detection antibodies, such that signals obtained from the different detectable elements can be distinguished. The nature of such detection steps is discussed elsewhere in this specification.

Any combination of detectable elements may be used in the present invention, provided it allows for generation of signals that can be distinguished and that do not interfere with each other (i.e. do not influence each other's measured value). The detectable elements may be detected simultaneously or subsequently to each other. A useful example of a suitable combination of detectable elements may be Europium, Terbium and Samarium, which can be detected by time-resolved fluorescence and produce distinct and non-interfering signals.

In an embodiment, the method comprises one or more steps which allow for quantitative measuring of the signals and comparing the measured signal intensities (i.e., signal intensities as obtained by the quantitative measuring) to references, whereby the concentrations in the sample of the *n* analytes and the total concentration of the *N* analytes are determined. The nature of such measurement steps and of calibration references is discussed elsewhere in this specification.

In an embodiment, the capture antibody is immobilized on a solid support. Immobilization and solid supports are discussed elsewhere in this specification.

In an embodiment, at least one of the capture or detection antibodies is a monoclonal antibody.

In another embodiment, the capture antibody and each detection antibody are monoclonal antibodies. Possible advantages of monoclonal antibodies are discussed elsewhere in this specification.

In a particularly useful embodiment, *N* equals 2 and n equals 1, the 2 analytes are insulin and proinsulin, the common portion being the A and B chains of insulin, the capture antibody is specific to an epitope within insulin, one detection antibody is specific for an epitope within insulin which is different from the epitope for which the capture antibody is specific, and the other detection antibody is specific to an epitope within C-peptide. This allows for determining the concentration in a sample of proinsulin and of molecules comprising insulin, from which the concentration of insulin and the proinsulin/insulin ratio can be computed.

Proinsulin and molecules comprising insulin whose concentrations are to be determined may be of either human or animal origin. In the latter case, proinsulin and molecules comprising insulin may originate, for example, from mouse, rat, dog, cat or pig, or from another animal species, and in particular such species commonly employed in laboratory practice. Accordingly, the sample in which the concentrations of proinsulin and molecules comprising insulin are to be determined may be obtained or derived from either a human or an animal (e.g., an animal as above, i.e., mouse, rat, dog, cat or pig, or another animal species, especially one commonly employed in laboratory practice), or if not directly obtained or derived from a human or an animal, may contain proinsulin and molecules comprising insulin with amino acid sequence corresponding to such molecules of human or animal origin.

The term "insulin" refers to human insulin, as well as to animal insulin. Insulin is formed by A and B chains, which typically comprise amino acids 66 to 86 of the above human proinsulin sequence or the corresponding part of an animal proinsulin sequence, and amino acids 1 to 30 of the above human proinsulin sequence or the corresponding part of an animal proinsulin sequence, respectively. The sequence of human insulin may be as above or may comprise amino acid substitutions due to normal genetic variation in human populations or due to pathogenic mutations. Usually, normal genetic variation will lead to no more than 10 amino acid substitutions and typically less than 5 amino acid substitutions. Similar considerations apply to animal insulin sequence. The A and B chains are typically linked by di-sulfide bonds between C19 and C85, between C71 and C76, and between C7 and C72 in human insulin, or between the corresponding C residues of animal insulin. Insulin will typically contain all three disulfide bonds but also encompasses insulin having any two of these bonds or only one of the C19-C85 or C7-C72 bonds in human insulin or the corresponding bonds in animal insulin. Human insulin may also encompass truncation products of the full-length insulin which may result from elimination of one or more amino acids from the free termini of the chains or from internal cleavage within the chains, for example, by action of non-specific proteases. Insulin further encompasses any modified (e.g., glycosylated, phosphorylated, or labeled) forms of full-length or truncated insulin.

Accordingly, the present invention also provides a method to determine the concentrations in a sample of proinsulin and of molecules comprising insulin, wherein the concentrations are determined within the same reaction compartment and using a capture antibody specific for an epitope within insulin, according to claim 16.

Because molecules comprising insulin in a physiologically relevant sample primarily include proinsulin and insulin, the difference between the concentration of these molecules and the concentration of proinsulin may be considered an estimate of the concentration of insulin. From these values the proinsulin/insulin ratio can be constructed.

The method of the present invention to determine the concentrations in a sample of proinsulin and of molecules comprising insulin, comprises:
(i.) binding of the capture antibody to molecules comprising insulin from the sample,
(ii.) binding of a detection antibody specific for an epitope within insulin, said epitope being different from the epitope for which the capture antibody is specific, to molecules comprising insulin from the sample, and
(iii.) binding of another detection antibody specific for an epitope within C-peptide to proinsulin from the sample,
wherein the binding takes place within the same reaction compartment.

In an embodiment, after the binding of the capture antibody and of the detection antibodies to their respective epitopes within proinsulin and molecules comprising insulin from the sample, essentially all proinsulin (i.e., essentially all proinsulin molecules) from the sample is bound by the capture antibody and by each detection antibody, and essentially all molecules comprising insulin from the sample are bound by the capture antibody and by at least the detection antibody specific for an epitope within insulin. Herein, "essentially all" refers to 80% or more, preferably 90% or more, more preferably 95% or more and most preferably 99% or more of the molecules.

The method for determining the concentrations in a sample of proinsulin and of molecules comprising insulin in fact represents a close equivalent of the method for determining the concentrations in a sample of proinsulin and of molecules comprising C-peptide, which has been extensively detailed elsewhere in this description. Therefore, a skilled person armed with the disclosed teachings regarding the latter method will immediately realize the preferred embodiments of the method for determining the concentrations in a sample of proinsulin and of molecules comprising insulin.

The specification also describes a kit-of-parts for use in the more general method described above.

The kit comprise:
(i.) a capture antibody specific for an epitope within the common portion present in the *N* analytes,
(ii.) a detection antibody specific for an epitope within the common portion present in the *N* analytes, said epitope being different from the epitope for which the capture antibody is specific,
(iii.) *n* detection antibodies, each specific for an epitope within the distinct portion of another of the *n* analytes,
wherein *N* is 2 or more and *n* is any integer value from 1 to *N*.

In an embodiment, *n* is *N*-1.

In another aspect, the invention provides a kit to determine the concentrations in a sample of proinsulin and of molecules comprising insulin, the kit comprising:
(i.) a capture antibody specific for an epitope within insulin,
(ii.) a detection antibody specific for an epitope within C-peptide, and
(iii.) another detection antibody specific for an epitope within insulin, said epitope being different from the epitope for which the capture antibody is specific.

In an embodiment the kit comprises antibodies specific for insulin and C-peptide of human origin.

In an embodiment the kit comprises antibodies specific for insulin and C-peptide of animal origin. In an embodiment such animal is mouse, rat, dog, cat or pig, or another animal species, and in particular such species commonly employed in laboratory practice.

A skilled person will realize that - apart from the choice of antibodies - specific embodiments of the above kit are largely equivalent to the embodiments of the kit for use in the method to determine in a sample the concentrations of proinsulin and molecules comprising C-peptide, as detailed elsewhere.

### EXAMPLES

**Example 1: Method precision comparison for proinsulin/C-peptide ratio determination.**

Plasma samples from the clinical routine were selected to obtain 3 pools of quality controls with different C-peptide concentrations. Each pool was analyzed 4 times with individual tests for proinsulin (as in Kjems et al. Clin Chem 1993; 39:2146-50) and C-peptide (using Time Resolved Fluorescence Immunoassay of AutoDELFIA kit of Perkin Elmer), and four times using the method according to the present invention to determine the concentrations of proinsulin and of molecules comprising C-peptide in the same reaction compartment and using a capture specific to an epitope within C-peptide. Proinsulin/C-peptide ratios were calculated for measurements obtained from the individual tests, as well as from the measurements obtained using the method of the invention. Standard deviations and corresponding coefficients of variation (CV %) were calculated.

### Protocol used in the method according to the present invention

### - antibodies

PEP-001 monoclonal antibody (Kjems et al. Clin Chem 1993;39:2146-50; Sobey et al. Biochem J 1989;260:535-41) was chosen as the capture antibody. This antibody recognizes an epitope in the C-terminal portion of C-peptide. CPT3F11 monoclonal antibody (Novo Nordisk) was chosen as the detection antibody specific for an epitope within C-peptide. This antibody recognizes an epitope in the N-terminal portion of C-peptide and is labeled with Europium (i.e., detectable element). HUI-001 anti-insulin monoclonal antibody (Kjems et al. Clin Chem 1993) was chosen at the detection antibody specific for an epitope within proinsulin, said epitope not being present in C-peptide. This antibody is labeled with biotin (i.e., detectable element).

### - coating of microtiter plates with the capture antibody

The concentration of stock of PEP 001 is 2,5 mg protein/mL. 1 part of PEP 001 stock (400 µL) is gently mixed with 3 parts of Gly-HCl 0.1 M, pH 2.5 (1200 µL), and incubated after mixing for 30 min at room temperature. To this mixture, 148.4 mL of coating buffer (0.2 M NaH₂PO₄, pH 4.5) is added to obtain 150 mL of coating solution with final dilution of antibody 1/375. 150 µL of the coating solution is added to each well of a microtiterplate and incubated overnight at 35 °C. Exemplary microtiter plates are maxisorp C96 (Nunc) or Delfia Yellow plates (Perkin-Elmer). Afterwards, wells are washed twice with "washing" solution prepared by diluting (in water) a 20x "washing" concentrate, which is prepared as follows:
6.06 g TRIS, 90 g NaCl, 0.45 mL Tween 20 (100 %), and 5.5 ml 6 N HCl are added to 400 mL water and pH is adjusted to exactly pH 7.75 with HCl, after which final volume is adjusted to exactly 500 mL water.

The wells are subsequently coated with sorbitol using "sorbitol coating" solution (50 mM NaH₂PO₄ /Na₂H PO₄ pH 7.0, 6 % sorbitol (w/V), 0.1% BSA (w/V)). 200 µL of this solution is added to each well of the microtiter plate, incubated overnight, afterwards aspirated, and the wells are allowed to dry. The thus prepared plates are kept dry in a dessicator.

### - calibration standards (reference)

The calibration standards contain both C-peptide and proinsulin standard molecules, with a molar proinsulin/C-peptide ratio of about 2% over the whole concentration range. The standards have been calibrated against the 1 st WHO International Reference Reagent IRR 84/510 for human C-peptide, and IRR 84/611 for proinsulin, both obtained from the NIBSC, South Mimms, U.K. Concentration of proinsulin and C-peptide in the standards series is as follows:

| Standard | [C-peptide] in pmol/L | [Proinsulin] in pmol/L |
|---|---|---|
| A | 0 | 0 |
| B | 133.74 | 2.8 |
| C | 668.69 | 14.0 |
| D | 1337.37 | 28 |
| E | 4012.12 | 84 |
| F | 8024.24 | 168 |

In order to obtain standards with similar properties as a plasma matrix, the calibrators are prepared in a buffer comparable to the Delfia diluent II (Perkin Elmer): 0.01 mol/L Tris-HCl buffer (1.211 g/L of Trizma base) with 9 g/L NaCl, 50 g/L BSA (pure for RIA), 2 mL tween/L and 0.1 % NaN3 (w/V) as preservative, pH 7.4 at room temperature. This buffer is also the calibrator "A". The standards series have been prepared by diluting a proinsulin standard stock (840 pmol /L) and a C-peptide standard stock (331.000 pmol/L) in the above buffer.

### - quality control (QC) samples

Plasma samples from the clinical routine were selected to obtain 3 pools of quality controls with different C-peptide concentrations of respectively 132.4, 472.0 and 1687 pmol/L C-peptide (measured preliminary as a control with the Perkin Elmer C-peptide TRFIA kit).

### - incubation of QC samples with the capture antibody

25 µL of standard calibrators A to F or 25 µL of a QC-sample was added per well of the coated microtiter plate and 100 µL of "PBS 3% BSA" buffer was added in each well. This buffer is made as follows:
Add to 500 mL of 0.04 mol/L Na₂HPO₄/NaH₂PO₄ phosphate buffer (pH 7.4) 30 g NaCl, 15 g BSA (low in heavy metals), 0.5 g gamma Globulin, 0.5 mL Tween 20, and 0.25 g/L NaN3 and dissolve; just before use add 1 mL of fetal bovine serum (0.1 µm sterile filtered) per 9 mL of this buffer.

After addition of the sample and the "PBS 3% BSA" buffer the microtiterplate plates were shaken at room temperature for 30 min and subsequently incubated overnight at 4°C without shaking. Each standard calibrator was assayed in double and each QC-sample was assayed four times in double.

### - washing step

After incubation with capture antibody, the wells were emptied and washed 4 times with "washing" solution.

### -incubation with the detection antibodies

300 µL of Eu-labeled CPT3F11 (30 mg/L) and 2 µl biotinylated HUI-001 stock (480 mg protein/L) were diluted in 13 mL of Assay buffer (50 mM Tris-HCl, pH 7.8, 9 g/L NaCl, 0.5 g/L NaN₃, 0.5g/L purified BSA (from Serologicals : BSA fraction V reagent grade code n° 81-066), 0.1 mL Tween 20, 7.4 mg/L EDTA, 200 mg/L anti-heterophyle antibody PolyMAK-33 from Roche diagnostic, 100 mg/L mouse immunoglobulin against HAMA from Scantibodies, France) and 125 µL of the solution were added to each well. The plate was shaken at room temperature for 30 minutes and incubated overnight at 4°C.

### - washing step

After incubation with detection antibodies, the wells were emptied and washed 4 times with "washing" solution.

### - incubation with streptavidin-Terbium

The HUI-001 antibody is labeled with biotin which needs to be further detected using labeled streptavidin (here labeled with Terbium). 13 mL of a 0.12 µg/mL streptavidin-Tb solution in assay buffer was prepared and 125 µL was added to each well. The plate was for 60 min at room temperature with shaking. Approximately 10 biotin molecules are conjugated per each HUI-001 antibody. This leads to an amplification of the proinsulin signal. In later detection steps, this partially compensates for the lower concentration of proinsulin in plasma compared to molecules comprising C-peptide and the approximately two times lower signal of Terbium compared to Europium. As mentioned elsewhere, Europium would have been preferred as a label for the detection antibody recognizing the less prevalent component, i.e., proinsulin. However, such antibody was unavailable to us at the time of conducting the above experiments.

### -washing step

After incubation with streptavidin-Tb, the wells were emptied and washed 4 times with "washing" solution.

### - fluorescence measurement

200 µL of "enhancement" solution (15 µM β-naphtoyltrifluoroacetone, 50µM tri-n-octylphosphine oxide, 0.1 % triton X-100 in 0.1 M acetone-potassium hydrogen phthalate buffer, pH 3.2; see Hemmilä et al. Anal. Biochem. 1984; 137:335-43) was added to each well and the plate was shaken at room temperature during 5 minutes. Eu fluorescence signal was measured on VICTOR Multilabel counter (Perkin Elmer) using settings recommended by the manufacturer. Afterwards, 50 µL of "enhancer C500-100" from Perkin Elmer (5 g/L Cetyltrimethylammonium bromide, 50 µM TMP-DPA, in 0.2 M Tris-acetate buffer, pH 11; see Hemmilä I et al. J. Biochem. Biophysic. Methods 1993, 26, 283-290) was added to each well, plates were shaken for stir 5 minutes and Tb signal was measured on VICTOR Multilabel counter (Perkin Elmer) using settings recommended by the manufacturer.

### - calculation

Concentrations of proinsulin and of molecules comprising C-peptide were obtained by comparing the measured signals from samples to calibration curves prepared by plotting the signals obtained from calibrator series against the known concentrations of proinsulin and C-peptide. The percentile form of the proinsulin/C-peptide ratio (PI/C %) was calculated as [(concentration of proinsulin)/(concentration of molecules comprising C-peptide *minus* concentration of proinsulin)]×100.

### Results

Standard deviations and coefficients of variance for proinsulin/C-peptide ratios in QC-samples determined using the method of the present invention (SD invention, CV% invention) and using individually measured concentrations of proinsulin and C-peptide (SD individual, CV% individual) is given in Table 1.

**Table 1.**

| Pool name | A | B | C | Mean A+B+C |
|---|---|---|---|---|
| Pl/C ratio % | 2.33 | 4.53 | 5.68 | |
| SD invention | 0.09 | 0.31 | 0.11 | - |
| SD individual | 0.65 | 0.83 | 1.00 | - |
| CV % invention | 6.40 | 9.07 | 2.56 | 6.01 |
| CV % individual | 19.63 | 15.24 | 14.39 | 16.42 |

Standard deviation and CV% for the determined proinsulin/C-peptide ratio are much smaller for the method of the present invention that for individual tests. Hence, the method of the present invention provides higher precision in the determination of proinsulin/C-peptide compared to the individual tests.

## Claims

1. A method to determine the concentrations in a sample of proinsulin and of molecules comprising C-peptide, wherein the concentrations are determined within the same reaction compartment and using a capture antibody specific for an epitope within C-peptide, the method comprising:
(i) binding of the capture antibody to molecules comprising C-peptide from the sample,
(ii) binding of a detection antibody specific for an epitope within C-peptide; said epitope being different from the epitope for which the capture antibody is specific, to molecules comprising C-peptide from the sample, and
(iii) binding of another detection antibody specific for an epitope within proinsulin, said epitope not being present in C-peptide, to proinsulin from the sample, wherein the binding takes place within the same reaction compartment.

2. The method according to claim 1, comprising one or more incubation steps allowing for the binding of the capture antibody and of the detection antibodies to their respective epitopes within proinsulin and molecules comprising C-peptide from the sample.

3. The method according to any of claims 1 or 2, wherein after the binding of the capture antibody and of the detection antibodies to their respective epitopes within proinsulin and molecules comprising C-peptide from the sample, essentially all proinsulin molecules from the sample are bound by the capture antibody and by both detection antibodies, and essentially all molecules comprising C-peptide from the sample are bound by the capture antibody and by at least the detection antibody specific for an epitope within C-peptide.

4. The method according to any of claims 1 to 3, comprising one or more washing steps to remove components of the sample which are not bound to the capture antibody or to remove detection antibodies which are not bound to their respective epitopes in proinsulin and in molecules comprising C-peptide from the sample.

5. The method according to any of claims 1 to 4, wherein each of the detection antibodies comprises a different detectable element.

6. The method according to claim 5, comprising one or more steps which allow for detecting the detectable elements comprised in the detection antibodies, such that signals obtained from the different detectable elements can be distinguished.

7. The method according to claim 6, comprising one or more steps which allow for quantitative measuring of the signals and comparing the measured signal intensities to references, whereby the concentrations in the sample of proinsulin and of molecules comprising C-peptide are determined.

8. The method according to any of claims 1 to 7, wherein the capture antibody is immobilized on a solid support.

9. A kit to determine the concentrations in a sample of proinsulin and of molecules comprising C-peptide, the kit comprising:
(i) a capture antibody specific for an epitope within C-peptide,
(ii) a detection antibody specific for an epitope within proinsulin, said epitope not being present in C-peptide, and
(iii) another detection antibody specific for an epitope within C-peptide, said epitope being different from the epitope for which the capture antibody is specific.

10. The kit according to claim 9, wherein the capture antibody is immobilized on a solid support.

11. The kit according to claim 9, wherein the capture antibody is immobilized within wells of a provided microtiter plate.

12. The kit according to any of claims 9 to 11, wherein each of the detection antibodies comprises a different detectable element.

13. The kit according to any of claims 9 to 12, wherein at least one of the capture and detection antibodies is a monoclonal antibody.

14. The kit according to claim 13, wherein the capture antibody and each of the detection antibodies are monoclonal antibodies.

15. The kit according to any of claims 9 to 14, wherein both detection antibodies are contained within the same container.

16. A method to determine the concentrations in a sample of proinsulin and of molecules comprising insulin, wherein the concentrations are determined within the same reaction compartment and using a capture antibody specific for an epitope within insulin, the method comprising:
(i) binding of the capture antibody to molecules comprising insulin from the sample,
(ii) binding of a detection antibody specific for an epitope within insulin, said epitope being different from the epitope for which the capture antibody is specific, to molecules comprising insulin from the sample, and
(iii) binding of another detection antibody specific for an epitope within C-peptide to proinsulin from the sample,
wherein the binding takes place within the same reaction compartment.

17. The method according to claim 16, comprising one or more incubation steps allowing for the binding of the capture antibody and of the detection antibodies to their respective epitopes within proinsulin and molecules comprising insulin from the sample.

18. The method according to any of claims 16 or 17, wherein after the binding of the capture antibody and of the detection antibodies to their respective epitopes within proinsulin and molecules comprising insulin from the sample, essentially all proinsulin molecules from the sample are bound by the capture antibody and by each detection antibody, and essentially all molecules comprising insulin from the sample are bound by the capture antibody and by at least the detection antibody specific for an epitope within insulin.

19. The method according to any of claims 16 to 18, comprising one or more washing steps to remove components of the sample which are not bound to the capture antibody or to remove detection antibodies which are not bound to their respective epitopes in proinsulin and in molecules comprising insulin from the sample.

20. The method according to any of claims 16 to 19, wherein each of the detection antibodies comprises a different detectable element.

21. The method according to claim 20, comprising one or more steps which allow for detecting the detectable elements comprised in the detection antibodies, such that signals obtained from the different detectable elements can be distinguished.

22. The method according to claim 21, comprising one or more steps which allow for quantitative measuring of the signals and comparing the measured signal intensities to references, whereby the concentrations in the sample of proinsulin and of molecules comprising insulin are determined.

23. The method according to any of claims 16 to 22, wherein the capture antibody is immobilized on a solid support.

24. A kit to determine the concentrations in a sample of proinsulin and of molecules comprising insulin, the kit comprising:
(i) a capture antibody specific for an epitope within insulin,
(ii) a detection antibody specific for an epitope within C-peptide, and
(iii) another detection antibody specific for an epitope within insulin, said epitope being different from the epitope for which the capture antibody is specific.

## Patentansprüche

1. Verfahren zur Ermittlung der Konzentrationen von Proinsulin und Molekülen, die C-Peptid umfassen, in einer Probe, wobei die Konzentrationen in demselben Reaktionskompartiment ermittelt werden und wobei ein Fangantikörper verwendet wird, der für ein Epitop im C-Peptid spezifisch ist, wobei das Verfahren das Folgende umfasst:
(i) Binden des Fangantikörpers an Moleküle aus der Probe, die C-Peptid umfassen,
(ii) Binden eines Detektionsantikörpers, der für ein Epitop im C-Peptid spezifisch ist, an Moleküle aus der Probe, die C-Peptid umfassen, wobei sich das Epitop von dem Epitop unterscheidet, für welches der Fangantikörper spezifisch ist,
(iii) Binden eines anderen Detektionsantikörpers, der für ein Epitop im Proinsulin spezifisch ist, an Proinsulin aus der Probe, wobei das Epitop im C-Peptid nicht vorliegt,
wobei die Bindung in demselben Reaktionskompartiment stattfindet.

2. Verfahren nach Anspruch 1, welches einen oder mehrere Inkubationsschritte umfasst, wobei die Bindung des Fangantikörpers und der Detektionsantikörper an ihre entsprechenden Epitope im Proinsulin aus der Probe und in Molekülen aus der Probe, die C-Peptid umfassen, ermöglicht wird.

3. Verfahren nach einem der Ansprüche 1 oder 2, wobei nach der Bindung des Fangantikörpers und der Detektionsantikörper an ihre entsprechenden Epitope im Proinsulin aus der Probe und in Molekülen aus der Probe, die C-Peptid umfassen, im Wesentlichen alle Proinsulinmoleküle aus der Probe von dem Fangantikörper und beiden Detektionsantikörpern gebunden sind und im Wesentlichen alle Moleküle aus der Probe, die C-Peptid umfassen, von dem Fangantikörper und zumindest von dem Detektionsantikörper, der für ein Epitop im C-Peptid spezifisch ist, gebunden sind.

4. Verfahren nach einem der Ansprüche 1 bis 3, welches einen oder mehrere Waschschritte umfasst, um Komponenten der Probe zu entfernen, welche nicht an den Fangantikörper gebunden sind, oder um Detektionsantikörper zu entfernen, welche nicht an ihre entsprechenden Epitope im Proinsulin und in Molekülen aus der Probe gebunden sind, die C-Peptid umfassen.

5. Verfahren nach einem der Ansprüche 1 bis 4, wobei jeder der Detektionsantikörper ein anderes detektierbares Element umfasst.

6. Verfahren nach Anspruch 5, welches einen oder mehrere Schritte umfasst, welche das Detektieren der detektierbaren Elemente ermöglichen, die in den Detektionsantikörpern enthalten sind, derart, dass Signale, die von den unterschiedlichen detektierbaren Elementen erhalten werden, unterschieden werden können.

7. Verfahren nach Anspruch 6, welches einen oder mehrere Schritte umfasst, welche eine quantitative Messung der Signale und einen Vergleich der gemessenen Signalintensitäten mit Referenzwerten ermöglichen, wodurch die Konzentrationen von Proinsulin und von Moleküle in der Probe, die C-Peptid enthalten, ermittelt werden können.

8. Verfahren nach einem der Ansprüche 1 bis 7, wobei der Fangantikörper auf einem festen Träger immobilisiert wird.

9. Testsatz zur Ermittlung der Konzentrationen von Proinsulin und Molekülen, die C-Peptid umfassen, in einer Probe, wobei der Testsatz das Folgende umfasst:
(i) einen Fangantikörper, der für ein Epitop im C-Peptid spezifisch ist,
(ii) einen Detektionsantikörper, der für ein Epitop im Proinsulin spezifisch ist, wobei das Epitop nicht im C-Peptid vorliegt,
(iii) einen anderen Detektionsantikörper, der für ein Epitop im C-Peptid spezifisch ist, wobei sich das Epitop von dem Epitop unterscheidet, für welches der Fangantikörper spezifisch ist.

10. Testsatz nach Anspruch 9, wobei der Fangantikörper auf einem festen Träger immobilisiert wird.

11. Testsatz nach Anspruch 9, wobei der Fangantikörper in Vertiefungen einer bereitgestellten Mikrotiterplatte immobilisiert wird.

12. Testsatz nach einem der Ansprüche 9 bis 11, wobei jeder der Detektionsantikörper ein anderes detektierbares Element umfasst.

13. Testsatz nach einem der Ansprüche 9 bis 11, wobei es sich bei mindestens einem der Fangantikörper und Detektionsantikörper um einen monoklonalen Antikörper handelt.

14. Testsatz nach Anspruch 13, wobei es sich bei dem Fangantikörper und bei jedem der Detektionsantikörper um monoklonale Antikörper handelt.

15. Testsatz nach einem der Ansprüche 9 bis 14, wobei beide Detektionsantikörper in demselben Behälter enthalten sind.

16. Verfahren zur Ermittlung der Konzentrationen von Proinsulin und Molekülen, die Insulin umfassen, in einer Probe, wobei die Konzentrationen in demselben Reaktionskompartiment ermittelt werden und wobei ein Fangantikörper verwendet wird, der für ein Epitop im Insulin spezifisch ist, wobei das Verfahren das Folgende umfasst:
(i) Binden des Fangantikörpers an Moleküle aus der Probe, die Insulin umfassen,
(ii) Binden eines Detektionsantikörpers, der für ein Epitop im Insulin spezifisch ist, an Moleküle aus der Probe, die Insulin umfassen, wobei sich das Epitop von dem Epitop unterscheidet, für welches der Fangantikörper spezifisch ist,
(iii) Binden eines anderen Detektionsantikörpers, der für ein Epitop im C-Peptid spezifisch ist, an Proinsulin aus der Probe,
wobei die Bindung in demselben Reaktionskompartiment stattfindet.

17. Verfahren nach Anspruch 16, welches einen oder mehrere Inkubationsschritte umfasst, wobei die Bindung des Fangantikörpers und der Detektionsantikörper an ihre entsprechenden Epitope im Proinsulin aus der Probe und in Molekülen aus der Probe, die Insulin umfassen, ermöglicht wird.

18. Verfahren nach einem der Ansprüche 16 oder 17, wobei nach der Bindung des Fangantikörpers und der Detektionsantikörper an ihre entsprechenden Epitope im Proinsulin aus der Probe und in Molekülen aus der Probe, die Insulin umfassen, im Wesentlichen alle Proinsulinmoleküle aus der Probe von dem Fangantikörper und jedem Detektionsantikörper gebunden sind und im Wesentlichen alle Moleküle aus der Probe, die Insulin umfassen, von dem Fangantikörper und zumindest von dem Detektionsantikörper, der für ein Epitop im Insulin spezifisch ist, gebunden sind.

19. Verfahren nach einem der Ansprüche 16 bis 18, welches einen oder mehrere Waschschritte umfasst, um Komponenten der Probe zu entfernen, welche nicht an den Fangantikörper gebunden sind, oder um Detektionsantikörper zu entfernen, welche nicht an ihre entsprechenden Epitope im Proinsulin und in Molekülen aus der Probe gebunden sind, die Insulin umfassen.

20. Verfahren nach einem der Ansprüche 16 bis 19, wobei jeder der Detektionsantikörper ein anderes detektierbares Element umfasst.

21. Verfahren nach Anspruch 20, welches einen oder mehrere Schritte umfasst, welche das Detektieren der detektierbaren Elemente ermöglichen, die in den Detektionsantikörpern enthalten sind, derart, dass Signale, die von den unterschiedlichen detektierbaren Elementen erhalten werden, unterschieden werden können.

22. Verfahren nach Anspruch 21, welches einen oder mehrere Schritte umfasst, welche eine quantitative Messung der Signale und einen Vergleich der gemessenen Signalintensitäten mit Referenzwerten ermöglichen, wodurch die Konzentrationen von Proinsulin und von Moleküle in der Probe, die Insulin enthalten, ermittelt werden können.

23. Verfahren nach einem der Ansprüche 16 bis 22, wobei der Fangantikörper auf einem festen Träger immobilisiert wird.

24. Testsatz zur Ermittlung der Konzentrationen von Proinsulin und Molekülen, die Insulin umfassen, in einer Probe, wobei der Testsatz das Folgende umfasst:
(i) einen Fangantikörper, der für ein Epitop im Insulin spezifisch ist,
(ii) einen Detektionsantikörper, der für ein Epitop im C-Peptid spezifisch ist,
(iii) einen anderen Detektionsantikörper, der für ein Epitop im Insulin spezifisch ist, wobei sich das Epitop von dem Epitop unterscheidet, für welches der Fangantikörper spezifisch ist.

## Revendications

1. Procédé de détermination des concentrations dans un échantillon de pro-insuline et de molécules comprenant le peptide C, les concentrations étant déterminées à l'intérieur du même compartiment de réaction et utilisant un anticorps de capture spécifique d'un épitope à l'intérieur du peptide C, le procédé comprenant :
(i) la liaison de l'anticorps de capture aux molécules comprenant le peptide C de l'échantillon,
(ii) la liaison d'un anticorps de détection spécifique d'un épitope à l'intérieur du peptide C, ledit épitope étant différent de l'épitope duquel l'anticorps de capture est spécifique, à des molécules comprenant le peptide C de l'échantillon, et
(iii) la liaison d'un autre anticorps de détection spécifique d'un épitope à l'intérieur de la pro-insuline, ledit épitope n'étant pas présent dans le peptide C, à la pro-insuline de l'échantillon, la liaison ayant lieu à l'intérieur du même compartiment de réaction.

2. Procédé selon la revendication 1, qui comprend une ou plusieurs étapes d'incubation permettant la liaison de l'anticorps de capture et des anticorps de détection à leurs épitopes respectifs à l'intérieur de la pro-insuline et des molécules comprenant le peptide C de l'échantillon.

3. Procédé selon l'une quelconque des revendications 1 ou 2, dans lequel après la liaison de l'anticorps de capture et des anticorps de détection à leurs épitopes respectifs à l'intérieur de la pro-insuline et des molécules comprenant le peptide C de l'échantillon, pratiquement toutes les molécules de pro-insuline de l'échantillon sont liées par l'anticorps de capture et par les deux anticorps de détection, et pratiquement toutes les molécules comprenant le peptide C de l'échantillon sont liées par l'anticorps de capture et par au moins l'anticorps de détection spécifique d'un épitope à l'intérieur du peptide C.

4. Procédé selon l'une quelconque des revendications 1 à 3, qui comprend une ou plusieurs étapes de lavage destinées à éliminer les composants de l'échantillon qui ne sont pas liés à l'anticorps de capture ou à éliminer les anticorps de détection qui ne sont pas liés à leurs épitopes respectifs dans la pro-insuline et dans les molécules comprenant le peptide C de l'échantillon.

5. Procédé selon l'une quelconque des revendications 1 à 4, dans lequel chacun des anticorps de détection comprend un élément détectable différent.

6. Procédé selon la revendication 5, qui comprend une ou plusieurs étapes permettant la détection des éléments détectables compris dans les anticorps de détection, de manière à ce que les signaux obtenus des différents éléments détectables puissent être distingués.

7. Procédé selon la revendication 6, qui comprend une ou plusieurs étapes permettant la mesure quantitative des signaux et la comparaison des intensités de signal mesurées à des références, moyennant quoi les concentrations dans l'échantillon de pro-insuline et de molécules comprenant le peptide C sont déterminées.

8. Procédé selon l'une quelconque des revendications 1 à 7, dans lequel l'anticorps de capture est immobilisé sur un support solide.

9. Kit de détermination des concentrations dans un échantillon de pro-insuline et de molécules comprenant le peptide C, le kit comprenant :
(i) un anticorps de capture spécifique d'un épitope à l'intérieur du peptide C,
(ii) un anticorps de détection spécifique d'un épitope à l'intérieur de la pro-insuline, ledit épitope n'étant pas présent dans le peptide C, et
(iii) un autre anticorps de détection spécifique d'un épitope à l'intérieur du peptide C, ledit épitope étant différent de l'épitope duquel l'anticorps de capture est spécifique.

10. Kit selon la revendication 9, dans lequel l'anticorps de capture est immobilisé sur un support solide.

11. Kit selon la revendication 9, dans lequel l'anticorps de capture est immobilisé à l'intérieur de puits d'une plaque de microtitration fournie.

12. Kit selon l'une quelconque des revendications 9 à 11, dans lequel chacun des anticorps de détection comprend un élément détectable différent.

13. Kit selon l'une quelconque des revendications 9 à 12, dans lequel au moins l'un des anticorps de capture et de détection est un anticorps monoclonal.

14. Kit selon la revendication 13, dans lequel l'anticorps de capture et chacun des anticorps de détection sont des anticorps monoclonaux.

15. Kit selon l'une quelconque des revendications 9 à 14, dans lequel les deux anticorps de détection sont contenus à l'intérieur du même récipient.

16. Procédé de détermination des concentrations dans un échantillon de pro-insuline et de molécules comprenant de l'insuline, les concentrations étant déterminées à l'intérieur du même compartiment de réaction et utilisant un anticorps de capture spécifique d'un épitope à l'intérieur de l'insuline, le procédé comprenant :
(i) la liaison de l'anticorps de capture à des molécules comprenant de l'insuline de l'échantillon,
(ii) la liaison d'un anticorps de détection spécifique d'un épitope à l'intérieur de l'insuline, ledit épitope étant différent de l'épitope duquel l'anticorps de capture est spécifique, à des molécules comprenant de l'insuline de l'échantillon, et
(iii) la liaison d'un autre anticorps de détection spécifique d'un épitope à l'intérieur du peptide C à la pro-insuline de l'échantillon,
la liaison ayant lieu à l'intérieur du même compartiment de réaction.

17. Procédé selon la revendication 16, qui comprend une ou plusieurs étapes d'incubation permettant la liaison de l'anticorps de capture et des anticorps de détection à leurs épitopes respectifs à l'intérieur de la pro-insuline et des molécules comprenant de l'insuline de l'échantillon.

18. Procédé selon l'une quelconque des revendications 16 ou 17, dans lequel après la liaison de l'anticorps de capture et des anticorps de détection à leurs épitopes respectifs à l'intérieur de la pro-insuline et des molécules comprenant de l'insuline de l'échantillon, pratiquement toutes les molécules de pro-insuline de l'échantillon sont liées par l'anticorps de capture et par chaque anticorps de détection, et pratiquement toutes les molécules comprenant de l'insuline de l'échantillon sont liées par l'anticorps de capture et par au moins l'anticorps de détection spécifique d'un épitope à l'intérieur de l'insuline.

19. Procédé selon l'une quelconque des revendications 16 à 18, qui comprend une ou plusieurs étapes de lavage destinées à éliminer les composants de l'échantillon qui ne sont pas liés à l'anticorps de capture ou à éliminer les anticorps de détection qui ne sont pas liés à leurs épitopes respectifs dans la pro-insuline et dans les molécules comprenant de l'insuline de l'échantillon.

20. Procédé selon l'une quelconque des revendications 16 à 19, dans lequel chacun des anticorps de détection comprend un élément détectable différent.

21. Procédé selon la revendication 20, qui comprend une ou plusieurs étapes permettant la détection des éléments détectables compris dans les anticorps de détection, de manière à ce que les signaux obtenus des différents éléments détectables puissent être distingués.

22. Procédé selon la revendication 21, qui comprend une ou plusieurs étapes permettant la mesure quantitative des signaux et la comparaison des intensités de signal mesurées à des références, moyennant quoi les concentrations dans l'échantillon de pro-insuline et de molécules comprenant de l'insuline sont déterminées.

23. Procédé selon l'une quelconque des revendications 16 à 22, dans lequel l'anticorps de capture est immobilisé sur un support solide.

24. Kit de détermination des concentrations dans un échantillon de pro-insuline et de molécules comprenant de l'insuline, le kit comprenant :
(i) un anticorps de capture spécifique d'un épitope à l'intérieur de l'insuline,
(ii) un anticorps de détection spécifique d'un épitope à l'intérieur d'un peptide C, et
(iii) un autre anticorps de détection spécifique d'un épitope à l'intérieur de l'insuline, ledit épitope étant différent de l'épitope duquel l'anticorps de capture est spécifique.
